# EUROPEAN PATENT APPLICATION

(11) **EP 4 306 039 A1**
(43) Date of publication of application: **17.01.2024**
(21) Application number: 21930476.3
(22) Date of filing: 02.11.2021
(51) Int. Cl.: A61B 5/00, A61B 5/053

(54) **DEMENTIA DIAGNOSIS METHOD AND SYSTEM USING BRAIN IMPEDANCE PATTERN**

(30) Priority: 09.03.2021 KR 20210030953
(71) Applicant: Megnosis, Inc., Daejeon 34046 (KR)
(72) Inventor: LEE, Soonhyouk, Daejeon 34046 (KR)
(74) Representative: V.O.
(86) International application number: PCT/KR2021/015655
(87) International publication number: WO 2022/191376

(57) **Abstract**

A dementia diagnosis method and system using a brain impedance pattern are disclosed. According to one embodiment, a dementia diagnosis system comprises at least one processor implemented to execute a computer-readable command, and the at least one processor can comprise: an electrode pair control unit for controlling a plurality of electrode pairs so that a current is introduced through each of the plurality of electrode pairs formed by two or more random electrodes from among electrodes attached to a subject to be diagnosed, the two or more random electrodes including electrodes attached in relation to the frontal lobe of the subject to be diagnosed; and a dementia diagnosis unit, which compares the magnitude of the impedance of each of the plurality of electrode pairs as a result of the current being introduced with a preset impedance value to diagnose whether dementia of the subject to be diagnosed has progressed.

## Description

### TECHNICAL FIELD

The following example embodiments relate to technology for a dementia diagnosis system and method using a brain impedance pattern.

### RELATED ART

Conventional dementia diagnosis technology detects scalp potential through a plurality of electrodes according to the International 10-20 standard for placement of electrodes and diagnoses whether a subject to be diagnosed has dementia using statistic on a time change of the average dipole degree obtained based on the detected potential. As described above, the conventional dementia diagnosis technology using statistic on the time change in the average dipole degree as a dementia degree parameter is described in Korean Patent Laid-Open Publication No. 10-2002-0048857.

However, the conventional dementia diagnosis technology has a disadvantage in that complexity of calculating a statistic on a time change of average dipole degree used as a dementia degree parameter is very high.

Therefore, there is a need for dementia diagnosis technology using an easily calculable dementia degree parameter.

### DETAILED DESCRIPTION

### TECHNICAL SUBJECT

Example embodiments provide a dementia diagnosis method and system using an easily calculable dementia degree parameter.

In detail, example embodiments provide a dementia diagnosis method and system using impedance of scalp potential detected through a plurality of electrodes according to the International 10-20 standard for placement of electrodes as a dementia degree parameter.

### TECHNICAL SOLUTION

According to an example embodiment, a dementia diagnosis system implemented as a computer system may include at least one processor configured to execute a computer-readable instruction. The at least one processor includes an electrode pair control unit configured to control a plurality of electrode pairs such that current is introduced through each of the plurality of electrode pairs formed by two or more random electrodes among electrodes attached to of a scalp a subject to be diagnosed, the two or more random electrodes including electrodes attached in relation to the frontal lobe of the subject to be diagnosed; and a dementia diagnosis unit configured to compare an impedance magnitude of each of the plurality of electrode pairs as an introduction result of the current to a preset impedance value and to diagnose whether the subject to be diagnosed has dementia.

According to an aspect, the dementia diagnosis unit may be configured to diagnose that the subject to be diagnosed has dementia when at least one electrode pair having the impedance magnitude greater than or equal to the preset impedance value is detected from among the plurality of electrode pairs.

According to another aspect, the dementia diagnosis unit may be configured to compare the impedance magnitude of each of the plurality of electrode pairs to the preset impedance value and to diagnose whether the subject to be diagnosed has dementia.

According to another aspect, the electrode pair control unit may be configured to sequentially switch the plurality of electrode pairs such that the current is introduced to one electrode pair at a time.

According to another aspect, the electrode pair control unit may be configured to control remaining electrode pairs excluding the electrode pair to which the current is introduced from among the plurality of electrode pairs such that the remaining electrode pairs are deactivated.

According to another aspect, the dementia diagnosis system may further include an electrode pair selector configured to form the plurality of electrode pairs by selecting the two or more random electrodes from among the electrodes attached to the subject to be diagnosed.

According to another aspect, the electrode pair selector may be configured to form a plurality of additional electrode pairs by selecting one electrode from among electrodes related to memory among the electrodes attached to the subject to be diagnosed and one random electrode from among the electrodes attached to the subject to be diagnosed, the electrode pair control unit may be configured to control the plurality of additional electrode pairs such that the current is introduced through the plurality of additional electrode pairs, and the dementia diagnosis unit may be configured to compare an impedance magnitude of each of the plurality of additional electrode pairs to the preset impedance value in at least one frequency and to diagnose a decrease in cognition and memory of the subject to be diagnosed.

According to another aspect, the dementia diagnosis unit may be configured to diagnose that the decrease in cognition and memory is present in the subject to be diagnosed when at least one electrode pair having the impedance magnitude greater than or equal to the preset impedance value is detected from among the plurality of additional electrode pairs in the at least one frequency.

According to another aspect, the electrode pair control unit may be configured to sequentially switch the plurality of additional electrode pairs such that the current is introduced to one additional electrode pair at a time.

According to another aspect, the electrode pair control unit may be configured to control remaining additional electrode pairs excluding the additional electrode pair to which the current is introduced from among the plurality of additional electrode pairs such that the remaining additional electrode pairs are deactivated.

According to another aspect, the dementia diagnosis system may further include a frequency converter configured to convert frequency of a signal; a constant current generator configured to generate constant current based on the signal; an electrode unit configured to include the plurality of electrode pairs and to introduce the current output from the constant current generator to scalp of the subject to be diagnosed using the electrode pair control unit included in the at least one processor; and a voltage detector configured to sequentially measure voltage for each of the plurality of electrode pairs to obtain the impedance magnitude of each of the plurality of electrode pairs using the dementia diagnosis unit included in the at least one processor and to deliver the measured voltage to the dementia diagnosis unit.

According to another aspect, the electrode unit may include a plurality of electrodes according to the International 10-20 standard for placement of electrodes.

According to an example embodiment, a dementia diagnosis method performed by a computer system may include controlling a plurality of electrode pairs such that current is introduced through each of the plurality of electrode pairs formed by two or more random electrodes among electrodes attached to a scalp of a subject to be diagnosed, the two or more random electrodes including electrodes attached in relation to the frontal lobe of the subject to be diagnosed; and comparing an impedance magnitude of each of the plurality of electrode pairs as an introduction result of the current to a preset impedance value and diagnosing whether the subject to be diagnosed has dementia.

According to an aspect, the diagnosing may include diagnosing that the subject to be diagnosed has dementia when at least one electrode pair having the impedance magnitude greater than or equal to the preset impedance value is detected from among the plurality of electrode pairs.

According to an example embodiment, in a computer program storing a computer-readable recording medium to implement a dementia diagnosis method in conjunction with a computer system, the dementia diagnosis method may include controlling a plurality of electrode pairs such that current is introduced through each of the plurality of electrode pairs formed by two or more random electrodes among electrodes attached to a scalp of a subject to be diagnosed, the two or more random electrodes including electrodes attached in relation to the frontal lobe of the subject to be diagnosed; and comparing an impedance magnitude of each of the plurality of electrode pairs as an introduction result of the current to a preset impedance value and diagnosing whether the subject to be diagnosed has dementia.

### EFFECT

Example embodiments may provide a dementia diagnosis method and system using an easily calculable dementia degree parameter.

In detail, example embodiments may provide a dementia diagnosis method and system using impedance of scalp potential detected through a plurality of electrodes according to the International 10-20 standard for placement of electrodes as a dementia degree parameter.

Therefore, example embodiments may provide a dementia diagnosis method and system that may minimize complexity and a calculation time of a dementia degree parameter.

### BRIEF DESCRIPTION OF DRAWINGS

FIG. 1 illustrates a dementia diagnosis system according to an example embodiment.
FIG. 2 illustrates an electrode unit included in a dementia diagnosis system according to an example embodiment.
FIG. 3 is a diagram illustrating an example of a component includable in a processor included in a dementia diagnosis system according to an example embodiment.
FIG. 4 is a flowchart illustrating a dementia diagnosis method according to an example embodiment.
FIGS. 5A and 5B illustrate impedance patterns for explaining a dementia diagnosis method according to an example embodiment.

### BEST MODE

Hereinafter, example embodiments will be described with reference to the accompanying drawings. However, the present invention is not limited to or restricted by the example embodiments. Also, like reference numerals provided in the respective drawings refer to like elements throughout.

Also, terminologies used herein are terms used to appropriately express example embodiments and may vary according to intention of a user or an operator or convention in the field to which the present invention pertains. Therefore, the terms need to be defined based on the contents throughout the present specification.

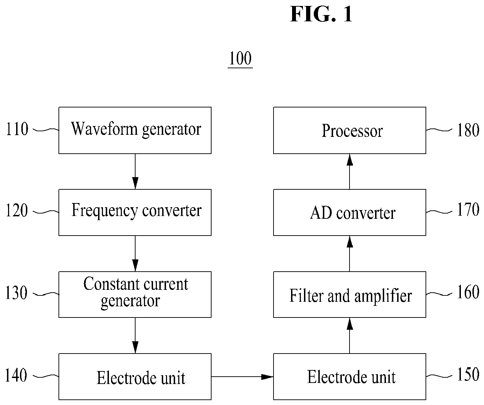

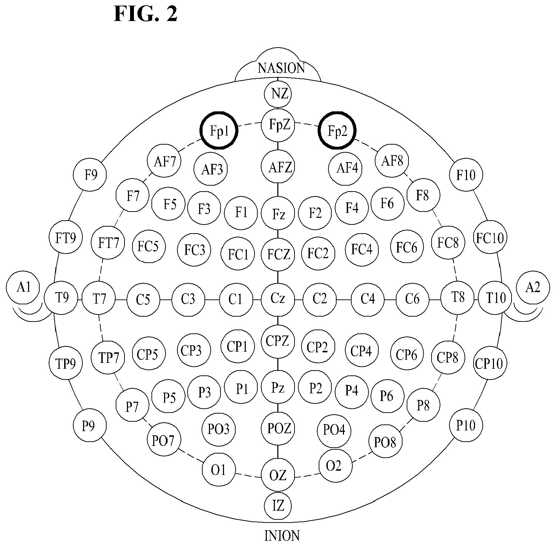

FIG. 1 illustrates a dementia diagnosis system according to an example embodiment, and FIG. 2 illustrates an electrode unit included in a dementia diagnosis system according to an example embodiment. A dementia diagnosis system 100 of FIG. 1 represents an example of including a waveform generator 110, a frequency converter 120, a constant current generator 130, an electrode unit 140, a voltage detector 150, a filter and amplifier 160, an analog-to-digital (AD) converter 170, and a processor 180. FIG. 1 is provided as an example only and the components of the dementia diagnosis system 100 are not limited to FIG. 1. For example, depending on example embodiments, the dementia diagnosis system 100 may be configured to omit the waveform generator 110 or the frequency converter 120.

The waveform generator 110 may generate a signal having at least one waveform. For example, the waveform generator 110 may generate a signal having at least one waveform among a trigonometric function (sine, cosine), a biphasic wave square wave or a wave in which direct current (DC) and alternating current (AC) are mixed, and pink noise.

The frequency converter 120 may make a signal having certain frequency that is output from the waveform generator 110. For example, the frequency converter 120 may change the frequency of the signal within the range of 0 hertz (Hz) to 200 megahertz (MHz).

The constant current generator 130 may generate constant current based on the signal of which the frequency is converted by the frequency converter 120. Current generated by the constant current generator 130 may have a value of 2 mA or less and may have a frequency of a low frequency band or a frequency of a high frequency band within the range of 0 Hz to 200 MHz.

Therefore, voltage for each of a plurality of electrode pairs measured by the voltage detector 150, which is described below, represents voltage in the low frequency band when the current generated by the constant current generator 130 has the frequency of the low frequency band and represents voltage in the high frequency band when the current generated by the constant current generator 130 has the frequency of the high frequency band.

The electrode unit 140 may be controlled by an electrode pair control unit included in the processor 180, which is described below, and may introduce current output from the constant current generator 130 to scalp of a subject to be diagnosed. In detail, the electrode unit 140 may be configured with a plurality of electrodes according to the International 10-20 standard for placement of electrodes as shown in FIG. 2 and may include a plurality of electrode pairs (e.g., FP1-F3, FP2-F4, FP1-P3, FP2-P4, FP1-F7, FP2-F8, FP1-C3, FP2-C4, FP1-T3, FP2-T4, FP1-T5, FP2-T6, etc.) formed by two or more random electrodes among electrodes attached to the subject to be diagnosed according to the International 10-20 standard for displacement of electrodes, to include the electrodes (e.g., FP1 and FP2) attached in relation to the frontal lobe of the subject to be diagnosed. In the following, although it is described that the plurality of electrode pairs are formed with two or more random electrodes, without being limited thereto or restricted thereby, the plurality of electrode pairs may be formed with two or more random electrodes (e.g., three electrodes or four electrodes). Also, the plurality of electrode pairs may include a randomly fixed one electrode pair and may include at least one electrode pair selected and thereby formed depending on cases. However, without being limited thereto or restricted thereby, all of the plurality of electrode pairs may be selected and thereby formed depending on cases.

Therefore, the electrode unit 140 may be controlled by the electrode pair control unit to allow current to flow in the plurality of electrode pairs. For example, in the electrode unit 140, the plurality of electrode pairs may be sequentially switched such that the current is introduced to one electrode pair at a time by the electrode pair control unit (e.g., at the same time at which the current is introduced to only an electrode pair of FP1-F3, remaining electrode pairs are deactivated, and at the same time at which the current is introduced to only an electrode pair of FP2-F4, remaining electrode pairs are deactivated). Here, the plurality of electrode pairs being sequentially switched represents that a sequential activation operation, such as one electrode pair being activated and remaining electrode pairs being deactivated, is performed. For example, a mechanical operation or an electrical switching operation, such as one electrode pair being in contact with the scalp of the subject to be diagnosed and the remaining electrode pairs being separated therefrom, may be sequentially performed for all of the plurality of electrode pairs. However, without being limited thereto or restricted thereby, the electrode pair control unit may allow the current to simultaneously flow in the plurality of electrode pairs or may allow the current to allow one random fixed electrode pair among the plurality of electrode pairs.

The voltage detector 150 may be controlled by a dementia diagnosis unit included in the processor 180, which is described below, and may measure scalp potential from the electrode unit 140 and may deliver the measured scalp potential to the dementia diagnosis unit through the filter and amplifier 160 and the AD converter 170. However, without being limited thereto or restricted thereby, the scalp potential measured by the voltage detector 150 may be delivered to the dementia diagnosis unit through the AD converter 170 alone. Therefore, that the voltage detector 150 measures the scalp potential and delivers the same to the dementia diagnosis unit represents that the voltage detector 150 measures the scalp potential and delivers the same to the dementia diagnosis unit through the AD converter 170 or (further through the filter and amplifier 160).

In detail, the voltage detector 150 may sequentially measure voltage for each of the plurality of electrode pairs and may deliver the measured voltage to the dementia diagnosis unit, such that the dementia diagnosis unit obtains an impedance magnitude of each of the plurality of electrode pairs (formed with two or more random electrodes among the electrodes attached to the subject to be diagnosed according to the International 10-20 standard of placement of electrodes to include electrodes attached in relation to the frontal lobe of the subject to be diagnosed).

Here, sequentially measuring the voltage for each of the plurality of electrode pairs may be performed at the same time at which the electrode unit 140 introduces the current while sequentially switching the plurality of electrode pairs. For example, immediately after the electrode unit 140 introduces the current by contacting one electrode pair to the scalp of the subject to be diagnosed, the voltage detector 150 may measure a scalp voltage using the corresponding electrode pair as is or using at least one separate electrode. This operational current introduction operation and voltage measurement operation may be sequentially performed for all of the plurality of electrode pairs. However, without being limited thereto or restricted thereby, the dementia diagnosis unit may simultaneously measure voltage for each of the plurality of electrode pairs or may measure voltage for one fixed random electrode pair among the plurality of electrode pairs.

The filter and amplifier 160 may filter out noise of a signal for the scalp potential output from the voltage detector 150 and may amplify the signal. The filter and amplifier 160 may be omitted depending on an implementation example.

The AD converter 170 may convert the scalp potential measured by the voltage detector to a digital signal and may deliver the converted digital signal to the processor 180 (in more detail, the dementia diagnosis unit included in the processor 180).

The processor 180 may be physically and functionally included in the dementia diagnosis system 100 and may also be independently implemented in physical terms and included only in functional terms. For example, some of components of the processor 180 may be included in an external terminal (not shown) configured to generate a dementia diagnosis request.

The processor 180 may be configured to process instructions of a computer program by performing basic arithmetic operations, logic operations, and input/output (I/O) operations. The instructions may be provided from a memory (not shown) or a communication program (not shown) to the processor 180. For example, the processor 180 may be configured to execute an instruction received according to a program code stored in a storage device, such as a memory.

Here, the memory (not shown) may include a permanent mass storage device such as random access memory (RAM), read only memory (ROM), and disk drive, as a computer-readable recording medium, connected to the processor 180. Also, an OS or at least one program code for an operation of the processor 180 or according to the operation thereof may be stored in the memory. Such software components may be loaded from another computer-readable recording medium separate from the memory. The separate computer-readable recording medium may include a computer-readable recording medium, for example, a floppy drive, a disk, a tape, a DVD/CD-ROM drive, and a memory card.

A communication module (not shown) may provide a function for communication between the processor 180 and an external server (not shown), an external computer (not shown), or an external terminal (not shown) through connection to the processor 180. For example, a dementia diagnosis request for the subject to be diagnosed may be delivered from the external server or the external computer to the processor 180 through a network (not shown) and the communication module. Inversely, data (e.g., dementia diagnosis result) generated according to an operation of the processor 180 may be transmitted to the external terminal through the communication module and the network.

The network (not shown) may be configured to support a near-distance communication scheme between devices as well as a communication scheme using a communication network (e.g., mobile communication network, wired Internet, wireless Internet, broadcasting network). For example, the network may include at least one network among a personal area network (PAN), a local area network (LAN), a campus area network (CAN), a metropolitan area network (MAN), a wide area network (WAN), a broadband network (BBN), the Internet, and the like. Also, the network may include at least one network topology that includes a bus network, a star network, a ring network, a mesh network, a star-bus network, a tree or hierarchical network, and the like. However, they are provided as examples only.

In particular, the processor 180 refers to an entity that performs the following dementia diagnosis method and may perform the dementia diagnosis method for diagnosing whether the subject to be diagnosed has dementia in response to a service request (dementia diagnosis request) generated through an I/O device (not shown) or the communication module included in the dementia diagnosis system 100.

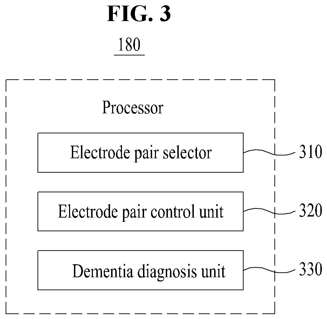

To this end, the processor 180 may include components to perform the dementia diagnosis method while being connected to the AD converter 170, the electrode unit 140, and the like in a wired manner or to the aforementioned network in a wireless manner. Further description related thereto is made with reference to FIG. 3 below.

Here, the I/O device (not shown) refers to a device configured to input data to the processor 180 or to output data processed by the processor 180. Here, an input device may include a device, such as a keyboard and a mouse, and an output device may include a device, such as a display. As another example, the I/O device may be a device in which an input function and an output function are integrated into one, such as a touchscreen.

Hereinafter, example embodiments of the dementia diagnosis method and system are described.

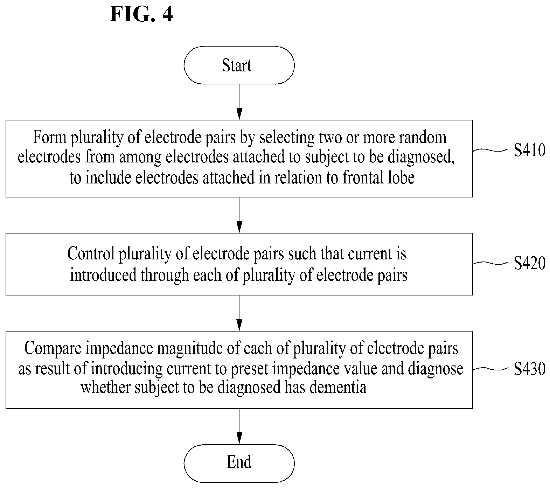

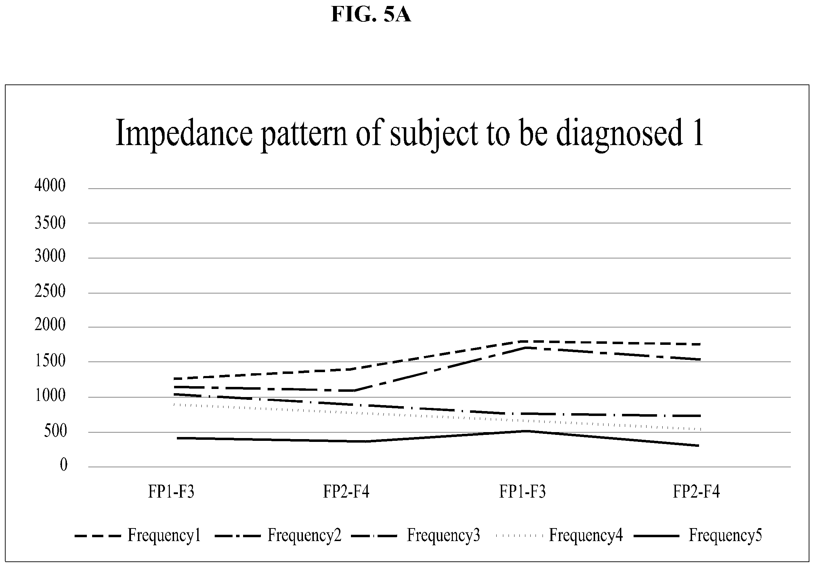

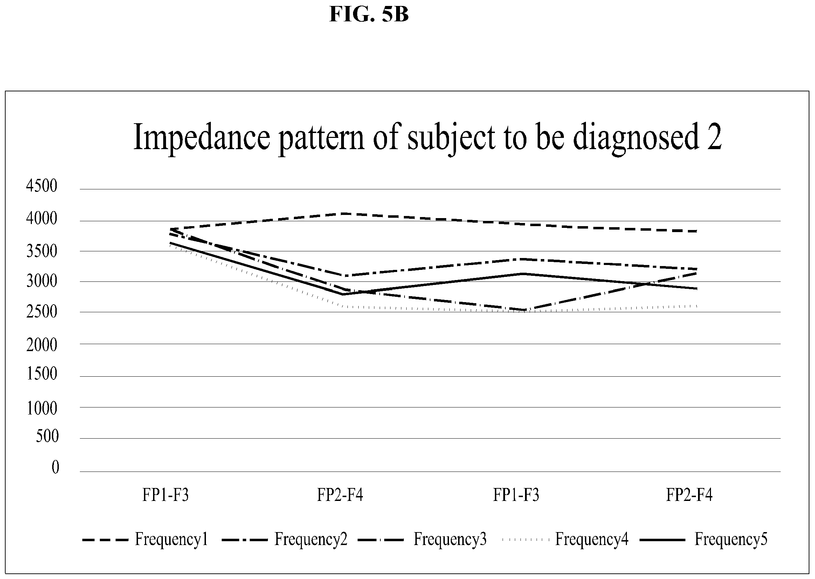

FIG. 3 is a diagram illustrating an example of a component includable in a processor included in a dementia diagnosis system according to an example embodiment, FIG. 4 is a flowchart illustrating a dementia diagnosis method according to an example embodiment, and FIGS. 5A and 5B illustrate impedance patterns for explaining a dementia diagnosis method according to an example embodiment.

The processor 180 according to an example embodiment may include key components of the dementia diagnosis system, that is, an electrode pair selector 310, an electrode pair control unit 320, and a dementia diagnosis unit 330 as illustrated in FIG. 3 and may perform the dementia diagnosis method of FIG. 4. Depending on example embodiments, the components of the processor 180 may be optionally included in or excluded from the processor 180. For example, by implementing the electrode pair control unit 320 to fixably use a plurality of electrode pairs formed with two or more random electrodes among electrodes attached to a scalp of a subject to be diagnosed, the electrode pair selector 310 may be omitted from the processor 180.

Also, depending on example embodiments, the components of the processor 180 may be separated or merged for functional representations of the processor 180. For example, at least a portion of the components of the processor 180 may be implemented on the external terminal that generates a dementia diagnosis request.

The components of the processor 180 may be implemented to execute an instruction according to a code of at least one program related to operations S410 to S430 included in the dementia diagnosis method.

Here, the components of the processor 180 may be representations of different functions of the processor 180 performed by the processor 180 in response to an instruction provided from at least one program code. For example, the electrode pair selector 310 may be used as a functional representation of the processor 180 that forms a plurality of electrode pairs by selecting two or more random electrodes from among the electrodes attached to the subject to be diagnosed, to include electrodes attached in relation to the frontal lobe of the subject to be diagnosed.

In operation S410, the electrode pair selector 310 may form a plurality of electrode pairs by selecting two or more random electrodes from among electrodes attached to a subject to be diagnosed, to include electrodes attached in relation to the frontal lobe of the subject to be diagnosed. Here, forming the plurality of electrode pairs by selecting the two or more random electrodes from among the electrodes attached to the subject to be diagnosed to include the electrodes attached in relation to the frontal lobe of the subject to be diagnosed represents selecting the two or more random electrodes from among the electrodes attached to the subject to be diagnosed, including the electrodes attached in relation to the frontal lobe of the subject to be diagnosed, and setting the plurality of electrode pairs to which current is to be introduced for dementia diagnosis.

For example, the electrode pair selector 310 may form the plurality of electrode pairs (FP1-F3, FP2-F4, FP1-P3, FP2-P4, FP1-F7, FP2-F8, FP1-C3, FP2-C4, FP1-T3, FP2-T4, FP1-T5, FP2-T6, etc.) by selecting the two or more random electrodes (here, the two or more random electrodes including the electrodes (FP1, FP2) attached in relation to the frontal lobe of the subject to be diagnosed) from among the plurality of electrodes according to the International 10-20 standard for displacement of electrodes as illustrated in FIG. 2.

In operation S420, the electrode pair control unit 320 may control the plurality of electrode pairs such that the current is introduced through each of the plurality of electrode pairs. In detail, the electrode pair control unit 320 may sequentially switch the plurality of electrode pairs such that the current is introduced to one electrode pair at a time for all of the plurality of electrode pairs. For example, the electrode pair control unit 320 may control the plurality of electrode pairs such that the current is introduced by contacting one electrode pair among the plurality of electrode pairs to the scalp of the subject to be diagnosed and remaining electrode pairs are deactivated and, in this manner, may minimize noise in an operation of measuring voltage for each of the plurality of electrode pairs. As described above, a switching operation (sequential activation operation) of the plurality of electrode pairs may be based on a mechanical operation or an electrical switching operation of the plurality of electrode pairs.

Here, in operation S420, the dementia diagnosis unit 330 may measure voltage (scalp voltage) for each of the plurality of electrode pairs through the voltage detector. In detail, at the same time at which the electrode pair control unit 320 sequentially switches the plurality of electrode pairs such that the current is introduced to one electrode pair for all of the plurality of electrode pairs, the dementia diagnosis unit 330 may sequentially measure voltage of the plurality of electrode pairs by measuring voltage of the electrode pair to which the current is introduced. For example, when the electrode pair control unit 320 introduces the current by contacting one electrode pair to the scalp of the subject to be diagnosed, the dementia diagnosis unit 330 may measure the scalp voltage using the corresponding electrode pair as is through the voltage detector or using at least one separate electrode. This operating current introduction operation and voltage measurement operation may be sequentially performed for all of the plurality of electrode pairs in operation S420.

Therefore, once operation S420 is performed, the dementia diagnosis unit 330 may obtain voltage for each of the plurality of electrode pairs and may calculate impedance of each of the plurality of electrode pairs used in operation S430. That is, although it is not illustrated in operation S420, operation S420 may include all of current introduction operation, voltage measurement operation, and impedance calculation operation.

Here, as described above, when the electrode pair selector 310 is omitted from the processor 180, the dementia diagnosis method according to an example embodiment may start from operation S420 without performing operation S410. In this case, in operation S420, the electrode pair control unit 320 may perform the aforementioned operation of controlling the plurality of electrode pairs fixably using the plurality of electrode pairs formed with the two or more random electrodes among the electrodes attached to the subject to be diagnosed (the two or more random electrodes including the electrodes attached in relation to the frontal lobe of the subject to be diagnosed).

In operation S430, the dementia diagnosis unit 330 may compare an impedance magnitude of each of the plurality of electrode pairs as a result of introducing the current to a preset impedance value and may diagnose whether the subject to be diagnosed has dementia. In detail, when at least one electrode pair having the impedance magnitude greater than or equal to the preset impedance value is detected from among the plurality of electrode pairs, the dementia diagnosis unit 330 may diagnose that the subject to be diagnosed has dementia. Hereinafter, the preset impedance value refers to a preset threshold and may represent not a maximum value but a specific value for determining dementia.

For example, the impedance value is proportional to a measured voltage value. Therefore, when a maximum measurement voltage AD converted value (impedance value) is determined as 4095 using the AD converter with a 12-bit resolution and a measurement voltage AD converted value (impedance value) of one electrode pair among the plurality of electrode pairs as a result of introducing the current is detected as 2050, there are an electrode pair having the voltage AD converted value (impedance value) of 2050 greater than or equal to a preset impedance value (2047) corresponding to a preset ratio of 50% compared to 4095 that is the maximum measurement voltage AD converted value. Accordingly, the dementia diagnosis unit 330 may diagnose that the subject to be diagnosed has dementia.

Here, the dementia diagnosis unit 330 may allow the current to be generated by the constant current generator 130 and to be introduced to the scalp through the plurality of electrode pairs and allow the voltage detector 150 to measure voltage of each of the plurality of electrode pairs and may compare an impedance magnitude of each of the plurality of electrode pairs to the preset impedance value based on the measured voltage in operation S430.

For example, referring to FIG. 5A, in the case of subject to be diagnosed 1, an impedance magnitude of each of the plurality of electrode pairs in a frequency band has a value less than 2000 that is less than a preset impedance value (2047) corresponding to 50% of 4095 that is a maximum impedance value. In this case, the dementia diagnosis unit 330 may diagnose that the subject to be diagnosed 1 does not have dementia. On the contrary, referring to FIG. 5B, in the case of subject to be diagnosed 2, an impedance magnitude of each of the plurality of electrode pairs in a frequency band has values greater than or equal to 2500 that is greater than or equal to the preset impedance value (2047) corresponding to 50% of 4095 that is the preset impedance value. In this case, the dementia diagnosis unit 330 may diagnose that the subject to be diagnosed 2 has dementia.

Also, the dementia diagnosis system 100 according to an example embodiment may additionally diagnose a decrease in cognition and memory of the subject to be diagnosed in addition to diagnosis of dementia. The diagnosis of the decrease in cognition and memory may be simultaneously performed with the aforementioned diagnosis of dementia. Alternatively, the diagnosis of the decrease in cognition and memory may be performed after the aforementioned diagnosis of dementia is completed and the subject to be diagnosed is diagnosed to have dementia.

For example, the electrode pair selector 310 may form a plurality of additional electrode pairs (e.g., F3-P3, F4-P4, P3-T3, P4-T4, F3-F7, F4-F8, P3-T5, P4-T6, etc.) by selecting one electrode from among electrodes (e.g., F3, F4, P3, P4) related to memory among the electrodes attached to the subject to be diagnosed and one random electrode from among the electrodes attached to the subject to be diagnosed in operation S410, the electrode pair control unit 320 may control the plurality of additional electrode pairs such that the current is introduced through the plurality of additional electrode pairs in operation S420, and the dementia diagnosis unit 330 may compare an impedance magnitude of each of the plurality of additional electrode pairs to the preset impedance value in at least one frequency and may diagnose a decrease in cognition and memory of the subject to be diagnosed in operation S430. In this manner, diagnosis of the decrease in cognition and memory may be simultaneously performed with diagnosis of dementia.

As another example, when the aforementioned diagnosis of dementia is performed through operations S410 to S430 and the subject to be diagnosed is diagnosed to have dementia, the electrode pair selector 310 may form the plurality of additional electrode pairs (e.g., F3-T3, F4-P4, P3-T3, P4-T4, F3-F7, F4-F8, P3-T5, P4-T6, etc.) by selecting one electrode from among the electrodes (e.g., F3, F4, P3, P4) related to memory among the electrodes attached to the subject to be diagnosed in an additional first operation (not shown) and the electrode pair control unit 320 may control the plurality of additional electrode pairs such that the current is introduced to the plurality of additional electrode pairs in an additional second operation (not shown). In this manner, the dementia diagnosis unit 330 may compare an impedance magnitude of each of the plurality of additional electrode pairs to preset impedance value in at least one frequency and may diagnose a decrease in cognition and memory of the subject to be diagnosed in an additional third operation (not shown).

In all of the aforementioned two examples, when at least one electrode pair having the impedance magnitude greater than or equal to the preset impedance value is detected from among the plurality of additional electrode pairs in at least one frequency, the dementia diagnosis unit 330 may diagnose that the subject to be diagnosed has the decrease in cognition and memory (e.g., when an electrode pair having the preset impedance value or more corresponding to 50% that is a preset ratio of a maximum impedance value is detected in at least one frequency, the dementia diagnosis unit 330 diagnoses that the subject to be diagnosed has the decrease in cognition and memory).

Also, in all of the aforementioned examples, similar to the dementia diagnosis process, the electrode pair control unit 320 may sequentially switch the plurality of additional electrode pairs using a mechanical method or an electrical switching method such that the current is introduced to one additional electrode pair at a time (e.g., the electrode pair control unit 320 controls remaining additional electrode pairs to activate the additional electrode pair to which the current is introduced and to deactivate the remaining additional electrode pairs among the plurality of additional electrode pairs).

Although the example embodiments describe a 2-terminal electrode method for introducing current through a plurality of electrode pairs formed using two or more random electrodes and measuring voltage to include electrodes attached in relation to the frontal lobe of a subject to be diagnosed, the example embodiments may also be applied as a 4-terminal electrode method.

In this case, the current may be introduced to current introduction electrode pairs formed using two or more random electrodes to include the electrodes attached in relation to the frontal lobe of the subject to be diagnosed, voltage may be measured through voltage measurement electrode pairs formed using the two or more random electrodes excluding the current introduction electrode pairs to which the current is introduced, and the aforementioned dementia diagnosis process may be performed based on the 4-terminal electrode method. Even in the 4-terminal electrode method, an impedance magnitude of each of the voltage measurement electrode pairs may be compared to the preset impedance value to perform a dementia diagnosis.

The apparatuses described herein may be implemented using hardware components, software components, and/or a combination thereof. For example, the apparatuses and the components described herein may be implemented using one or more general-purpose or special purpose computers, such as, for example, a processor, a controller, an arithmetic logic unit (ALU), a digital signal processor, a microcomputer, a field programmable gate array (FPGA), a programmable logic unit (PLU), a microprocessor, or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will be appreciated that a processing device may include multiple processing elements and/or multiple types of processing elements. For example, a processing device may include multiple processors or a processor and a controller. In addition, different processing configurations are possible, such as parallel processors.

The software may include a computer program, a piece of code, an instruction, or some combinations thereof, for independently or collectively instructing or configuring the processing device to operate as desired. Software and/or data may be embodied in any type of machine, component, physical equipment, a computer storage medium or device, to be interpreted by the processing device or to provide an instruction or data to the processing device. The software also may be distributed over network coupled computer systems so that the software is stored and executed in a distributed fashion. The software and data may be stored by one or more computer readable storage media.

The methods according to the above-described example embodiments may be configured in a form of program instructions performed through various computer devices and recorded in computer-readable media. Here, the media may continuously store computer-executable programs or may temporarily store the same for execution or download. Also, the media may be various types of recording devices or storage devices in a form in which one or a plurality of hardware components are combined and may be present over a network in a distributed manner without being limited to media directly connected to a computer system. Examples of the media include magnetic media such as hard disks, floppy disks, and magnetic tapes; optical media such as CD-ROM and DVDs; magneto-optical media such as floptical disks; and hardware devices that are specially configured to store program instructions, such as read-only memory (ROM), random access memory (RAM), flash memory, and the like. Examples of other media may include recording media and storage media managed by an app store that distributes applications or a site, a server, and the like that supplies and distributes other various types of software.

While the example embodiments are described with reference to specific example embodiments and drawings, it will be apparent to one of ordinary skill in the art that various alterations and modifications in form and details may be made in these example embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other example embodiments, and equivalents of the claims are to be construed as being included in the claims.

## Claims

1. A dementia diagnosis system implemented as a computer system, the dementia diagnosis system comprising:
at least one processor configured to execute a computer-readable instruction,
wherein the at least one processor comprises:
an electrode pair control unit configured to control a plurality of electrode pairs such that current is introduced through each of the plurality of electrode pairs formed by two or more random electrodes among electrodes attached to a scalp of a subject to be diagnosed, the two or more random electrodes including electrodes attached in relation to the frontal lobe of the subject to be diagnosed; and
a dementia diagnosis unit configured to compare an impedance magnitude of each of the plurality of electrode pairs as an introduction result of the current to a preset impedance value and to diagnose whether the subject to be diagnosed has dementia.

2. The dementia diagnosis system of claim 1, wherein the dementia diagnosis unit is configured to diagnose that the subject to be diagnosed has dementia when at least one electrode pair having the impedance magnitude greater than or equal to the preset impedance value is detected from among the plurality of electrode pairs.

3. The dementia diagnosis system of claim 1, wherein the dementia diagnosis unit is configured to compare the impedance magnitude of each of the plurality of electrode pairs to the preset impedance value and to diagnose whether the subject to be diagnosed has dementia.

4. The dementia diagnosis system of claim 1, wherein the electrode pair control unit is configured to sequentially switch the plurality of electrode pairs such that the current is introduced to one electrode pair at a time.

5. The dementia diagnosis system of claim 4, wherein the electrode pair control unit is configured to control remaining electrode pairs excluding the electrode pair to which the current is introduced from among the plurality of electrode pairs such that the remaining electrode pairs are deactivated.

6. The dementia diagnosis system of claim 1, further comprising:
an electrode pair selector configured to form the plurality of electrode pairs by selecting the two or more random electrodes from among the electrodes attached to the subject to be diagnosed.

7. The dementia diagnosis system of claim 6, wherein the electrode pair selector is configured to form a plurality of additional electrode pairs by selecting one electrode from among electrodes related to memory among the electrodes attached to the subject to be diagnosed and one random electrode from among the electrodes attached to the subject to be diagnosed,
the electrode pair control unit is configured to control the plurality of additional electrode pairs such that the current is introduced through the plurality of additional electrode pairs, and
the dementia diagnosis unit is configured to compare an impedance magnitude of each of the plurality of additional electrode pairs to the preset impedance value in at least one frequency and to diagnose a decrease in cognition and memory of the subject to be diagnosed.

8. The dementia diagnosis system of claim 7, wherein the dementia diagnosis unit is configured to diagnose that the decrease in cognition and memory is present in the subject to be diagnosed when at least one electrode pair having the impedance magnitude greater than or equal to the preset impedance value is detected from among the plurality of additional electrode pairs in the at least one frequency.

9. The dementia diagnosis system of claim 7, wherein the electrode pair control unit is configured to sequentially switch the plurality of additional electrode pairs such that the current is introduced to one additional electrode pair at a time.

10. The dementia diagnosis system of claim 9, wherein the electrode pair control unit is configured to control remaining additional electrode pairs excluding the additional electrode pair to which the current is introduced from among the plurality of additional electrode pairs such that the remaining additional electrode pairs are deactivated.

11. The dementia diagnosis system of claim 1, further comprising:
a frequency converter configured to make a signal having certain frequency;
a constant current generator configured to generate constant current based on the signal;
an electrode unit configured to include the plurality of electrode pairs and to introduce the current output from the constant current generator to scalp of the subject to be diagnosed using the electrode pair control unit included in the at least one processor; and
a voltage detector configured to sequentially measure voltage for each of the plurality of electrode pairs to obtain the impedance magnitude of each of the plurality of electrode pairs using the dementia diagnosis unit included in the at least one processor and to deliver the measured voltage to the dementia diagnosis unit.

12. The dementia diagnosis system of claim 11, wherein the electrode unit includes a plurality of electrodes according to the International 10-20 standard for placement of electrodes.

13. A dementia diagnosis method performed by a computer system, the dementia diagnosis method comprising:
controlling a plurality of electrode pairs such that current is introduced through each of the plurality of electrode pairs formed by two or more random electrodes among electrodes attached to a subject to be diagnosed, the two or more random electrodes including electrodes attached in relation to the frontal lobe of the subject to be diagnosed; and
comparing an impedance magnitude of each of the plurality of electrode pairs as an introduction result of the current with a preset impedance value and diagnosing whether the subject to be diagnosed has dementia.

14. The dementia diagnosis method of claim 13, wherein the diagnosing comprises diagnosing that the subject to be diagnosed has dementia when at least one electrode pair having the impedance magnitude greater than or equal to the preset impedance value is detected from among the plurality of electrode pairs.

15. A computer program storing a computer-readable recording medium to implement a dementia diagnosis method in conjunction with a computer system, the dementia diagnosis method comprising:
controlling a plurality of electrode pairs such that current is introduced through each of the plurality of electrode pairs formed by two or more random electrodes among electrodes attached to a subject to be diagnosed, the two or more random electrodes including electrodes attached in relation to the frontal lobe of the subject to be diagnosed; and
comparing an impedance magnitude of each of the plurality of electrode pairs as an introduction result of the current with a preset impedance value and diagnosing whether the subject to be diagnosed has dementia.
